(19) Europäisches Patentamt
European Patent Office
Office européen des brevets

(11) **EP 2 690 083 B1**

(12) **EUROPÄISCHE PATENTSCHRIFT**

(45) Veröffentlichungstag und Bekanntmachung des Hinweises auf die Patenterteilung:
**10.08.2016 Patentblatt 2016/32**

(51) Int Cl.:
*C07C 41/09* ^(2006.01)     *C10L 1/02* ^(2006.01)

(21) Anmeldenummer: **13003164.4**

(22) Anmeldetag: **21.06.2013**

(54) **Verfahren und Vorrichtung zur Umwandlung eines Alkohols in ein Kraftstoffgemisch**

Method and apparatus for converting an alcohol into a fuel mixture

Procédé et dispositif de conversion d'un alcool en carburant mixte

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.07.2012 DE 102012014755**

(43) Veröffentlichungstag der Anmeldung:
**29.01.2014 Patentblatt 2014/05**

(73) Patentinhaber: **MAN Truck & Bus AG**
**80995 München (DE)**

(72) Erfinder: **Döring, Andreas**
**82008 München (DE)**

(56) Entgegenhaltungen:
**WO-A1-01/02515     US-A- 4 422 412**
**US-A- 4 876 989     US-A1- 2006 180 099**

EP 2 690 083 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

Die Erfindung betrifft ein Verfahren zur Umwandlung eines Alkohols in ein Kraftstoffgemisch gemäß dem Oberbegriff des Patentanspruchs 1 sowie eine Vorrichtung zu dessen Durchführung gemäß dem Patentanspruch 10.

Die Substitution konventioneller fossiler Kraftstoffe durch alternative Kraftstoffe gewinnt immer stärker an Bedeutung. In diesem Zusammenhang sind vor allem Alkohole, wie Methanol oder Ethanol, interessant, da sie relativ einfach aus regenerativen Quellen hergestellt werden können. Allerdings können diese Kraftstoffe aufgrund ihrer geringen Cetanzahl nicht in selbstzündenden Dieselmotoren verwendet werden. Um eine dieselmotorische Verbrennung zu ermöglichen, können diesen Kraftstoffen Additive, wie Dimethylether oder Diethylether, zur Anhebung der Cetanzahl zugegeben werden.

Ein Gemisch aus Alkohol, Ether und Wasser kann auch direkt während des Betriebs einer Brennkraftmaschine in einem vorgelagerten Prozessschritt aus Alkohol, beispielsweise aus Methanol oder Ethanol, mit Hilfe eines geeigneten Katalysators hergestellt werden.

US 4 876 989 A beschreibt eine Vorrichtung sowie ein Verfahren zur Leistungssteigerung eines mit Alkohol betriebenen Motors während kalter Betriebsbedingungen. Die Leistungssteigerung ist auf eine Etherzeugung zurückzuführen, welche während kalter Betriebsbeding sowohl das Starten des Motors und/oder das Erreichen reduzierter Abgasemissionen gewährleistet.

WO 01/02515 A1 lehrt ein Verfahren zum Betreiben eines Dieselmotors das eine kontinuierliche Alkohol zu Ether und Wasser Dehydration vorseht. Diese Stoffe werden direkt in den Brennraum injiziert um die Verbrennungstemperatur zu senken und damit die Stickstoffkonzentration im Abgas zu reduzieren.

Aus der US 44 22 412 A ist ein Verfahren zur Umwandlung von Alkohol in ein Kraftstoffgemisch bekannt, bei dem mittels eines Katalysators in einem Reaktor der auf eine geeignete Reaktionstemperatur erwärmte Alkohol, beispielsweise Methanol, in ein Kraftstoffgemisch gemäß beiliegender Reaktionsgleichung umgewandelt wird:

$$2\ CH_3OH \leftrightarrow CH_3\text{-}O\text{-}CH_3 + H_2O \qquad \text{Reaktion 1}$$

Mit dem hier verwendeten Methanol ($CH_3OH$) entsteht somit zusätzlich im Reaktor Dimethylether ($CH_3\text{-}O\text{-}CH_3$) und Wasser ($H_2O$). Wird dagegen Ethanol zur Herstellung des Kraftstoffgemischs für das Umwandlungsverfahren verwendet, kann mittels des Reaktors ein Gemisch aus Ethanol, Diethylether und Wasser hergestellt werden. Als Katalysator kann dabei im Reaktor Gamma-Aluminium eingesetzt werden.

Das gemäß diesem bekannten Verfahren hergestellte Kraftstoffgemisch unterliegt sehr starken Qualitätsschwankungen, was durch einen stark schwankenden Wasseranteil in Erscheinung tritt, weshalb eine Verwendung für den dieselmotorischen Betrieb in der Praxis nicht möglich ist. Auftretende Qualitätsschwankungen führen zu stark schwankenden Cetanzahlen und in deren Folge zu zeitlich stark schwankenden Zündzeitpunkten. Außerdem ist das sich ergebende Kraftstoffgemisch nicht immer für alle Motorbetriebspunkte gleichermaßen geeignet.

Der Erfindung liegt die Aufgabe zugrunde, ein Verfahren zur Umwandlung eines Alkohols in ein Kraftstoffgemisch für eine Brennkraftmaschine anzugeben, mit dem eine möglichst gleichbleibende Qualität des sich ergebenden Kraftstoffgemischs erzielt werden kann.

Die Lösung dieser Aufgabe erhält man durch die im Patentanspruchs 1 angegebenen Merkmale. Besonders vorteilhafte Weiterbildungen des erfindungsgemäßen Verfahrens sind in den Unteransprüchen 2 bis 8 offenbart.

Gemäß Patentanspruch 1 wird das Mischungsverhältnis von Alkoholanteil, Etheranteil und Wasseranteil im Kraftstoffgemisch durch Steuerung wenigstens eines Reaktionsparameters eingestellt wobei die Steuerung wenigstens eines Reaktionsparameters in Abhängigkeit von Betriebsparametern eines Nachbehandlungssystems erfolgt. Das Mischungsverhältnis des sich aus der Umwandlung des Alkohols ergebenden Kraftstoffgemischs ist unter anderem von der Reaktionstemperatur abhängig, weshalb durch eine entsprechende Einstellung der Reaktionstemperatur eine Einstellung des Mischungsverhältnisses möglich ist. Die nachfolgende Tabelle zeigt beispielhaft die Abhängigkeit des Mischungsverhältnisses von der Reaktionstemperatur bei einem Reaktordruck von 2Mpa bei der Verwendung von Methanol als Alkohol, der in einem Reaktor in ein Kraftstoffgemisch aus Alkohol, Ether und Wasser umgewandelt wird:

| Reaktionstemperatur [°C] | Alkoholanteil [%] | Etheranteil [%] | Wasseranteil [%] |
|---|---|---|---|
| 200 | 8 | 67 | 25 |
| 300 | 14 | 62 | 24 |
| 400 | 19 | 59 | 22 |
| 500 | 22 | 57 | 21 |

Die Einstellung einer gewünschten Reaktionstemperatur kann durch eine entsprechende Regelung der Temperatur des zugeführten Alkohols, beispielsweise des zugeführten Methanols, erfolgen. Ein weiterer Reaktionsparameter mit dem das Mischungsverhältnis des erfindungsgemäß hergestellten Kraftstoffgemisch eingestellt werden kann, ist der Reaktionsdruck.

Bei Untersuchungen hat sich gezeigt, dass die Bildung von Dimethylether (DME) gemäß Reaktion 1 folgendem Geschwindigkeitsansatz folgt:

$$-\frac{dc_{DME}}{dt} = \frac{k_r \, K_M^2 p_M^2}{(1 + K_M \, p_M)^2}$$

mit: $\dfrac{dc_{DME}}{dt}$ : *Stoffmengenänderungsgeschwindikeit von DME*

$K_M$ : *Gleichgewichstkonstante von Methanol*

$p_M$: *Methanol Partialdruck*

$k_r$ : *Geschwindigkeitskonstante der Reaktion*

$$k_r = k_0 \, e^{-\frac{E_A}{RT}} \, (Arrhenius - Ansatz) \quad E_A : Aktivierungsenergie \, (ca. \frac{60kJ}{mol})$$

Dies macht deutlich, dass die Geschwindigkeit, in der DME gebildet wird, zum einen vom Methanolpartialdruck, als auch, über die Temperaturabhängigkeit der Geschwindigkeitskonstanten $k_r$, von der Reaktionstemperatur abhängt.

Durch die Einstellung des Mischungsverhältnisses von Alkoholanteil, Etheranteil und Wasseranteil im Kraftstoffgemisch kann eine gewünschte Cetanzahl durch verhältnismäßig einfache Steuer- und Regelsysteme erreicht werden. Um eine Brennkraftmaschine im Betriebsbereich homogener Kompressionszündung betreiben zu können, kann eine Cetanzahl von unter 50 und/oder eine Oktanzahl von über 95 ROZ erreicht werden, während für den dieselmotorischen Betrieb ein Kraftstoffgemisch mit einer Cetanzahl von mehr als 50 durch eine entsprechende Steuerung der im Reaktor ablaufenden Reaktion erzeugt werden kann.

Die Steuerung wenigstens eines Reaktionsparameters kann insbesondere auch in Abhängigkeit von Betriebsparametern der Brennkraftmaschine erfolgen, für die das Kraftstoffgemisch vorgesehen ist. Damit kann das Kraftstoffgemisch in seinen Eigenschaften, beispielsweise an Betriebsparameter, wie Ladedruck, Drehzahl und/oder Last, angepasst werden, um einen optimierten Betrieb der Brennkraftmaschine zu erreichen.

Die Reaktionsparameter werden in Abhängigkeit von Betriebsparametern eines Abgasnachbehandlungssystems gesteuert, um beispielsweise eine möglichst schadstoffarme Verbrennung in der Brennkraftmaschine zu erhalten.

Die Reaktionsparameter können außerdem auch in Abhängigkeit von der Beschaffenheit des verwendeten Alkohols erfolgen, wobei auch die Reinheit des verwendeten Alkohols als Reaktionsparameter berücksichtigt werden kann.

Zur Umwandlung eines Alkohols in ein Kraftstoffgemisch kann beispielsweise Methanol oder Ethanol verwendet werden, wobei aber auch zur Erzielung gewünschter Kraftstoffeigenschaften ein Methanol-Ethanol-Gemisch als umzuwandelnder Alkohol verwendet werden kann. Das mit dem erfindungsgemäßen Verfahren hergestellte Kraftstoffgemisch kann demzufolge als Etheranteil Dimethylether und/oder Diethylether enthalten.

Der Erfindung liegt außerdem die Aufgabe zugrunde, eine Vorrichtung zur Umwandlung eines Alkohols in ein Kraftstoffgemisch für eine Brennkraftmaschine anzugeben, mit der das Kraftstoffgemisch bzgl. seiner Gemischanteile variiert werden kann.

Die Lösung der sich auf die Vorrichtung beziehenden Aufgabe wird durch die Merkmale des Patentanspruchs 9 erhalten. Besonders vorteilhafte Weiterbildungen der erfindungsgemäßen Vorrichtung sind in den Unteransprüchen 10 bis 14 offenbart.

Bei der Vorrichtung gemäß dem Patentanspruch 9 wird der dem Reaktor zugeführte Alkohol mittels einer Erwärmungseinrichtung erwärmt. Gemäß dem kennzeichnenden Teil des Patentanspruchs 9 ist eine Steuereinrichtung vorgesehen, die den Druck im Reaktor in Abhängigkeit von Reaktionsparametern einer im Reaktor ablaufenden Reaktion einstellt wobei die Steuerung wenigstens eines Reaktionsparameters in Abhängigkeit von Betriebsparametern eines Abgasnachbehandlungssystems ausführbar ist.

Zusätzlich zu dem Einstellen des Drucks im Reaktor in Abhängigkeit von Reaktionsparametern einer im Reaktor ablaufenden Reaktion kann vorzugsweise die Erwärmung des Alkohols in der Erwärmungseinrichtung in Abhängigkeit von Reaktionsparametern eines im Reaktor ablaufenden Umwandlungsprozesses und/oder von Betriebsparametern einer zugehörigen Brennkraftmaschine einstellbar sein. Durch eine entsprechende Erwärmung des zugeführten Alkohols ergeben sich unterschiedliche Reaktionstemperaturen, die beispielsweise in einem Bereich zwischen 200 und 500 °C variiert werden können. Je nach eingestellter Reaktionstemperatur erhält man als Reaktionsprodukt ein Kraftstoffgemisch mit einem gewünschten Alkohol-Ether-Wasser-Mischungsverhältnis. Das Mischungsverhältnis bestimmt maßgeblich die Verbrennungseigenschaften des Kraftstoffgemischs in der Brennkraftmaschine. Diese Verbrennungseigenschaften lassen sich auf einfache Weise durch eine Regelung der Reaktionstemperatur einstellen.

Die bevorzugte Ausführungsform der erfindungsgemäßen Vorrichtung besitzt eine Erwärmungseinrichtung, die aus einem Abgaswärmetauscher und einem Luftwärmetauscher besteht. Wenigstens einer der beiden Wärmetauscher wird bzgl. der Wärmeübertragung auf den hindurchströmenden Alkohol, der dem Reaktor zugeführt wird, gesteuert. Dabei dient der Luftwärmetauscher vorzugsweise als Kühlvorrichtung, um den im vorgeschalteten Abgaswärmetauscher erwärmten Alkohol auf eine gewünschte Prozesstemperatur abzukühlen. Dabei kann es ausreichend sein, nur die Kühlung durch den Luftwärmetauscher zu steuern, um eine gewünschte Reaktionstemperatur im Reaktor zu erhalten.

Mittels Sensoren, die die Temperatur und/oder den Druck im Reaktor messen, kann eine Steuereinrichtung als Teil eines Regelsystems die Erwärmung des zugeführten Alkohols steuern. Ein solches Temperaturregelsystem ist mit verhältnismäßig einfachen Mitteln realisierbar.

Zudem kann der Druck im Reaktor auch unabhängig von der Temperatur, beispielsweise mit Hilfe eines Druckregelventils und/oder durch Variation der zu- und/oder abgeführten Menge, geregelt bzw. verändert werden.

Der im Reaktor ablaufende Reaktionsprozess kann auch in Abhängigkeit vom Zylinderdruck und/oder in Abhängigkeit von Klopfsensorsignalen erfolgen. Zu diesem Zweck können Zylinderdrucksensoren und/oder Klopfsensoren an der Brennkraftmaschine angebracht sein, die über Messleitungen ihre Messsignale an die Steuereinrichtung zur Einstellung der Erwärmung des Alkohols und/oder des Drucks im Reaktor übertragen.

In der Steuereinrichtung, die die Erwärmung des dem Reaktor zugeführten Alkohols steuert, werden Betriebsparameter des Abgasnachbehandlungssystems berücksichtigt, um die Kraftstoffverbrennung zu optimieren. Insbesondere kann damit eine Reduzierung von Schadstoffen und auch eine verbesserte Kraftstoffverwertung erzielt werden.

Die Erfindung wird nachfolgend anhand eines in der Zeichnungsfigur 1 dargestellten Ausführungsbeispiels näher erläutert.

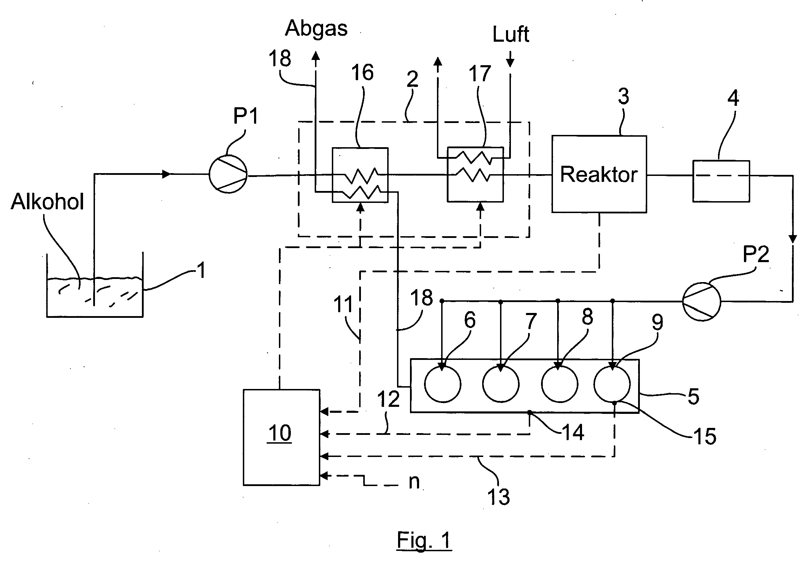

Figur 1 zeigt eine schematische Darstellung einer Vorrichtung zur Umwandlung von Alkohol in ein Kraftstoffgemisch, welches für den Betrieb einer Brennkraftmaschine als Kraftstoff dient.

Die Vorrichtung besitzt einen Tank 1, in welchem sich als Alkohol beispielsweise Methanol oder Ethanol befindet, der mittels einer ersten Pumpe P1 über eine Erwärmungseinrichtung 2 einem Reaktor 3 zugeführt wird. Im Reaktor 3 erfolgt eine Umwandlung des Alkohols bei einer geeigneten Reaktionstemperatur in ein Kraftstoffgemisch, welches über eine Kammer 4 und eine zweite Pumpe P2 der Brennkraftmaschine 5 zugeführt wird. An der Brennkraftmaschine 5 sind hierzu Einspritzdüsen 6 bis 9 angeordnet, über die das Kraftstoffgemisch in die Brennkammern der Brennkraftmaschine 5 eingespritzt wird.

Die in Figur 1 dargestellte Vorrichtung kann insbesondere in einem Kraftfahrzeug angeordnet sein, dessen Brennkraftmaschine 5 ein Dieselmotor ist.

Damit das der Brennkraftmaschine 5 zugeführte Kraftstoffgemisch ein für die Brennkraftmaschine 5 geeignetes Mischungsverhältnis der Anteile von Alkohol, Ether und Wasser hat, wird der Alkohol, bevor er dem Reaktor 3 zugeführt wird, auf eine hierfür geeignete Temperatur in der Erwärmungseinrichtung 2 erwärmt. Den Erwärmungsvorgang steuert eine Steuereinrichtung 10 in Abhängigkeit von mehreren Betriebsparametern. Die Steuereinrichtung 10 erhält hierzu von im Reaktor 3 und an der Brennkraftmaschine 5 vorgesehenen Sensoren über Messsignalleitungen 11 bis 13 Messsignale, die unterschiedliche Betriebsparameter repräsentieren. Aus dem Reaktor 3 erhält die Steuereinrichtung 10 die Reaktionstemperatur und den Reaktionsdruck als Messsignale von denen das Mischungsverhältnis von Alkohol, Ether und Wasser im Kraftstoffgemisch abhängt. Außerdem erhält die Steuereinrichtung 10 von einem Klopfsensor 14 und einem Zylinderdrucksensor 15 entsprechende Messsignale, die zusammen mit weiteren Betriebszustandssignalen, wie beispielsweise Drehzahl n, Last und Beschleunigung, den aktuellen Betriebszustand der Brennkraftmaschine 5 kennzeichnen. In Abhängigkeit von diesen Betriebsparametern der Brennkraftmaschine 5 kann die Steuereinrichtung 10 ein geeignetes Mischungsverhältnis für das Kraftstoffgemisch bestimmen und eine entsprechende Regelung der Reaktion im Reaktor 3 vornehmen.

Beim dargestellten Ausführungsbeispiel besteht die Erwärmungseinrichtung 2 aus einem Abgaswärmetauscher 16 und einem Luftwärmetauscher 17. Durch den Abgaswärmetauscher 16 führt die Abgasleitung 18 der Brennkraftmaschine 5, die im Abgaswärmetauscher 16 den ebenfalls durch den Abgaswärmetauscher 16 geleiteten Alkohol erwärmt. Der erwärmte Alkohol gelangt aus dem Abgaswärmetauscher 16 zum Luftwärmetauscher 17, wo mittels hindurchströmender Umgebungsluft der Alkohol auf eine gewünschte Temperatur abgekühlt werden kann. Sowohl der Abgaswärmetauscher 16 als auch der Luftwärmetauscher 17 können steuerbare Wärmetauscher sein, die eine Steuerung der Wärmeübertragung ermöglichen. So kann beispielsweise im Abgaswärmetauscher 16 durch eine Bypassregelung nur ein mehr oder weniger großer Anteil des Abgasstroms zur Erwärmung des hindurchströmenden Alkohols eingesetzt werden. Bei dem Luftwärmetauscher 17 kann zur geregelten Kühlung des hindurchströmenden Alkohols zum Beispiel der Luftdurchsatz durch den Luftwärmetauscher 17 von der Steuereinrichtung 10 gesteuert werden.

Selbstverständlich kann für den Wärmetauscher 17 an Stelle von Luft auch ein anderes Wärmeträgermedium, wie Wasser oder Öl, (hier nicht dargestellt) eingesetzt werden.

Neben der Temperatur kann auch der Druck im Reaktor mit Hilfe von Steuerorganen variiert werden. Zum einen kann dies durch Veränderung der von der Pumpe P1 geförderten Methanolmenge, beispielsweise durch Veränderung von Drehzahl oder Hub oder mit Hilfe einer auf der Saugseite der Pumpe P1 angeordneten variablen Drossel, erfolgen. Eine weitere Möglichkeit besteht in der Verwendung eines Druckregelventils auf der Druckseite der Pumpe P1, mit dessen Hilfe der Druck im Reaktor unabhängig von der Reaktortemperatur eingestellt werden kann.

Das im Reaktor 3 erzeugte Kraftstoffgemisch kann in einer dem Reaktor 3 nachgeschalteten Kammer 4 nachbehandelt oder zwischengelagert werden, soweit dies erforderlich ist.

## Patentansprüche

1. Verfahren zur Umwandlung eines Alkohols in ein aus Alkohol, Ether und Wasser bestehendes Kraftstoffgemisch, das zum Betrieb einer Brennkraftmaschine (5), insbesondere eines Verbrennungsmotors in einem Kraftfahrzeug, geeignet ist, wobei der Alkohol bei einer geeigneten Reaktionstemperatur in einem Reaktor (3) in das Kraftstoffgemisch umgewandelt wird, wobei das Mischungsverhältnis von Alkoholanteil, Etheranteil, und Wasseranteil im Kraftstoffgemisch durch Steuerung wenigstens eines Reaktionsparameters einer in dem Reaktor (3) ablaufenden Reaktion eingestellt wird, **dadurch gekennzeichnet, dass** die Steuerung wenigstens eines Reaktionsparameters in Abhängigkeit von Betriebsparametern eines Abgasnachbehandlungssystems erfolgt.

2. Verfahren nach Anspruch 1, **dadurch gekennzeichnet, dass** das Mischungsverhältnis durch Steuerung der Reaktionstemperatur und/oder des Reaktionsdrucks im Reaktor eingestellt wird.

3. Verfahren nach einem der Ansprüche 1 oder 2, **dadurch gekennzeichnet, dass** die Steuerung wenigstens eines Reaktionsparameters in Abhängigkeit von Betriebsparametern der Brennkraftmaschine (5) erfolgt.

4. Verfahren nach einem der vorhergehenden Ansprüche, **dadurch gekennzeichnet, dass** die Steuerung wenigstens eines Reaktionsparameters in Abhängigkeit von der Beschaffenheit des umzuwandelnden Alkohols erfolgt.

5. Verfahren nach einem der vorhergehenden Ansprüche, **dadurch gekennzeichnet, dass** für das Verfahren ein Methanol-Ethanol-Gemisch als umzuwandelnder Alkohol verwendet wird.

6. Verfahren nach einem der vorhergehenden Ansprüche, **dadurch gekennzeichnet, dass** das verfahrensgemäß hergestellte Kraftstoffgemisch als Etheranteil Dimethylether und/oder Diethylether enthält.

7. Verfahren nach einem der vorhergehenden Ansprüche, **dadurch gekennzeichnet, dass** die Brennkraftmaschine (5) im Betriebsbereich homogener Kompressionszündung betrieben wird.

8. Verfahren nach einem der Ansprüche 1 bis 7, **dadurch gekennzeichnet, dass** die Umwandlung des Alkohols in ein Kraftstoffgemisch so erfolgt, dass die Cetanzahl des Kraftstoffgemischs über 50 liegt.

9. Vorrichtung mit einem Reaktor (3), der mittels einer Erwärmungseinrichtung (2) erwärmten Alkohol in ein Kraftstoffgemisch für den Betrieb einer Brennkraftmaschine (5) umwandelt, zur Durchführung des Verfahrens nach Anspruch 1, **dadurch gekennzeichnet, dass**

   • eine Steuereinrichtung (10) den Druck im Reaktor in Abhängigkeit von Reaktionsparametern einer im Reaktor (3) ablaufenden Reaktion einstellt und
   • dass die Steuerung wenigstens eines Reaktionsparameters in Abhängigkeit von Betriebsparametern eines Abgasnachbehandlungssystems ausführbar ist.

10. Vorrichtung nach Anspruch 9, **dadurch gekennzeichnet, dass** die Erwärmungseinrichtung (2) aus einem Abgaswärmetauscher (16) zur Erwärmung und gegebenenfalls wenigstens einem zweiten Wärmetauscher (17), mit Luft oder Flüssigkeiten als Übertragungsmedium, zur Kühlungbesteht, und dass wenigstens in einem der beiden Wärmetauscher (16, 17) die Wärmeübertragung auf den hindurchströmenden Alkohol mittels der Steuereinrichtung (10) steuerbar ist.

11. Vorrichtung nach einem der Ansprüche 9 oder 10, **dadurch gekennzeichnet, dass** der zweite Wärmetauscher (17) zwischen dem Abgaswärmetauscher (16) und dem Reaktor (3) angeordnet ist und mittels der Umgebungsluft oder einer Flüssigkeit, insbesondere Wasser oder Öl, den im Abgaswärmetauscher (16) erwärmten Alkohol auf einen für die im Reaktor (3) ablaufende Reaktion geeigneten Temperatur- und/oder Druckwert kühlt.

12. Vorrichtung nach einem der Ansprüche 9 bis 11, **dadurch gekennzeichnet, dass** Sensoren die Temperatur und/oder den Druck im Reaktor (3) messen und die Messwerte als Messsignale an die Steuereinrichtung (10) übertragen.

13. Vorrichtung nach einem der Ansprüche 9 bis 12, **dadurch gekennzeichnet, dass** ein oder mehrere Zylinderdrucksensoren (15) und/oder ein oder mehrere Klopfsensoren (14) an der Brennkraftmaschine (5) vorgesehen sind, die ihre Messsignale an die Steuereinrichtung (10) als Betriebsparameter der Brennkraftmaschine (5) zur Einstellung der Erwärmung des umzuwandelnden Alkohols und/oder des Drucks im Reaktor übertragen.

14. Vorrichtung nach einem der Ansprüche 9 bis 13, **dadurch gekennzeichnet, dass** die Steuereinrichtung (10) die Drehzahl und die Last der Brennkraftmaschine (5) als Betriebsparameter bei der Einstellung der Erwärmung des umzuwandelnden Alkohols und/oder des Drucks im Reaktor berücksichtigt.

**Claims**

1. A method of converting an alcohol into a fuel mixture which consists of alcohol, ether and water and is suitable for operating a combustion engine (5), in particular an internal combustion engine in a motor vehicle, wherein the alcohol is converted into the fuel mixture in a reactor (3) at a suitable reaction temperature, wherein the mixing ratio of alcohol fraction, ether fraction and water fraction in the fuel mixture is adjusted by controlling at least one reaction parameter of a reaction taking place in the reactor (3), **characterized in that** at least one reaction parameter is controlled according to operating parameters of an exhaust-gas aftertreatment system.

2. The method according to Claim 1, **characterized in that** the mixing ratio is adjusted by controlling the reaction temperature and/or the reaction pressure in the reactor.

3. The method according to either Claim 1 or 2, **characterized in that** at least one reaction parameter is controlled according to operating parameters of the combustion engine (5).

4. The method according to any preceding claim, **characterized in that** at least one reaction parameter is controlled according to the constitution of the alcohol to be converted.

5. The method according to any preceding claim, **characterized in that** a methanol-ethanol mixture is used for the method as the alcohol to be converted.

6. The method according to any preceding claim, **characterized in that** the fuel mixture obtained according to said method contains dimethyl ether and/or diethyl ether as ether fraction.

7. The method according to any preceding claim, **characterized in that** the combustion engine (5) is operated in the operating region of homogeneous compression ignition.

8. The method according to any of Claims 1 to 7, **characterized in that** the alcohol is converted into a fuel mixture such that the cetane number of the fuel mixture is above 50.

9. An apparatus comprising a reactor (3) which converts an alcohol heated by means of a heating device (2) into a fuel mixture for operating a combustion engine (5), for practising the method according to Claim 1, **characterized in that**

   • a control device (10) adjusts the pressure in the reactor according to reaction parameters of a reaction taking place in the reactor (3) and
   • at least one reaction parameter is controllable according to operating parameters of an exhaust-gas aftertreatment system.

10. The apparatus according to Claim 9, **characterized in that** the heating device (2) consists of an exhaust-gas heat exchanger (16) for heating and optionally at least a second heat exchanger (17), with air or liquids as transfer medium, for cooling, and **in that** in at least one of the two heat exchangers (16, 17) the heat transfer to the alcohol flowing therethrough is controllable by means of the control device (10).

11. The apparatus according to either Claim 9 or 10, **characterized in that** the second heat exchanger (17) is disposed between the exhaust-gas heat exchanger (16) and the reactor (3) and uses the ambient air or a liquid, especially water or oil, to cool the alcohol heated in the exhaust-gas heat exchanger (16) to a temperature and/or pressure value which is suitable for the reaction taking place in the reactor (3).

12. The apparatus according to any of Claims 9 to 11, **characterized in that** sensors measure the temperature and/or pressure in the reactor (3) and transmit the measured values as measurement signals to the control device (10).

13. The apparatus according to any of Claims 9 to 12, **characterized in that** one or more cylinder pressure sensors (15) and/or one or more knocking sensors (14) are provided on the combustion engine (5) and transmit their measurement signals to the control device (10) as operating parameters of the combustion engine (5) to adjust the heating of the alcohol to be converted and/or the pressure in the reactor.

14. The apparatus according to any of Claims 9 to 13, **characterized in that** the speed and load of the combustion engine (5) are taken into account by the control device (10) as operating parameters in adjusting the heating of the alcohol to be converted and/or the pressure in the reactor.

## Revendications

1. Procédé pour la transformation d'un alcool en un mélange de carburant, constitué par de l'alcool, de l'éther et de l'eau, qui convient pour le fonctionnement d'un moteur à combustion interne (5), en particulier d'un moteur à combustion interne dans un véhicule automobile, l'alcool étant transformé à une température de réaction appropriée dans un réacteur (3) en ledit mélange de carburant, le rapport de mélange de la proportion d'alcool, de la proportion d'éther et de la proportion d'eau dans le mélange de carburant étant réglé par régulation d'au moins un paramètre de réaction d'une réaction qui se déroule dans le réacteur (3), **caractérisé en ce que** la régulation d'au moins un paramètre de réaction a lieu en fonction de paramètres de fonctionnement d'un système de posttraitement des gaz d'échappement.

2. Procédé selon la revendication 1, **caractérisé en ce que** le rapport de mélange est régulé par régulation de la température de réaction et/ou de la pression de réaction dans le réacteur.

3. Procédé selon l'une quelconque des revendications 1 ou 2, **caractérisé en ce que** la régulation d'au moins un paramètre de réaction a lieu en fonction des paramètres de fonctionnement du moteur .à combustion interne (5).

4. Procédé selon l'une quelconque des revendications précédentes, **caractérisé en ce que** la régulation d'au moins un paramètre de réaction a lieu en fonction de la qualité de l'alcool à transformer.

5. Procédé selon l'une quelconque des revendications précédentes, **caractérisé en ce qu'**on utilise, pour le procédé, un mélange méthanol-éthanol comme alcool à transformer.

6. Procédé selon l'une quelconque des revendications précédentes, **caractérisé en ce que** le mélange de carburant préparé selon le procédé contient, comme proportion d'éther, du diméthyléther et/ou du diéthyléther.

7. Procédé selon l'une quelconque des revendications précédentes, **caractérisé en ce que** le moteur à combustion interne (5) est exploité dans la plage de fonctionnement de l'allumage par compression homogène.

8. Procédé selon l'une quelconque des revendications 1 à 7, **caractérisé en ce que** la transformation de l'alcool en un mélange de carburant a lieu de manière telle que l'indice de cétane du mélange de carburant est supérieur à 50.

9. Dispositif présentant un réacteur (3), qui transforme de l'alcool réchauffé au moyen d'un dispositif de réchauffement

**EP 2 690 083 B1**

(2) en un mélange de carburant pour le fonctionnement d'un moteur à combustion interne (5), pour la réalisation du procédé selon la revendication 1, **caractérisé**

- **en ce qu'**un dispositif de régulation (10) règle la pression dans le réacteur en fonction de paramètres de réaction d'une réaction qui se déroule dans le réacteur (3) et
- **en ce que** la régulation d'au moins un paramètre de réaction peut être réalisée en fonction de paramètres de fonctionnement d'un système de posttraitement des gaz d'échappement.

10. Dispositif selon la revendication 9, **caractérisé en ce que** le dispositif de réchauffement (2) est constitué par un échangeur thermique à gaz d'échappement (16) pour le réchauffement et le cas échéant par au moins un deuxième échangeur thermique (17), fonctionnant avec de l'air ou des liquides comme milieu de transfert, pour le refroidissement et **en ce que** dans au moins un des deux échangeurs thermiques (16, 17), le transfert thermique à l'alcool qui s'écoule au travers de ceux-ci peut être réglé au moyen du dispositif de régulation (10).

11. Dispositif selon l'une quelconque des revendications 9 ou 10, **caractérisé en ce que** le deuxième échangeur thermique (17) est disposé entre l'échangeur thermique à gaz d'échappement (16) et le réacteur (3) et refroidit, au moyen de l'air environnant ou d'un liquide, en particulier de l'eau ou de l'huile, l'alcool réchauffé dans l'échangeur thermique à gaz d'échappement (16) à une valeur de température et/ou de pression appropriée pour la réaction qui se déroule dans le réacteur (3).

12. Dispositif selon l'une quelconque des revendications 9 ou 11, **caractérisé en ce que** des capteurs mesurent la température et/ou la pression dans le réacteur (3) et transmettent les valeurs de mesure comme signaux de mesures au dispositif de régulation (10).

13. Dispositif selon l'une quelconque des revendications 9 à 12, **caractérisé en ce qu'**il existe un ou plusieurs capteurs de pression (15) de cylindre et/ou un ou plusieurs capteurs de cliquetis (14) sur le moteur à combustion interne (5), qui transmettent leurs signaux de mesures au dispositif de régulation (10) comme paramètres de fonctionnement du moteur à combustion interne (5) pour le réglage du réchauffement de l'alcool à transformer et/ou de la pression dans le réacteur.

14. Dispositif selon l'une quelconque des revendications 9 à 13, **caractérisé en ce que** le dispositif de régulation (10) tient compte de la vitesse de rotation et de la charge du moteur à combustion interne (5) comme paramètres de fonctionnement lors du réglage du réchauffement de l'alcool à transformer et/ou de la pression dans le réacteur.

Fig. 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 4876989 A
- WO 0102515 A1
- US 4422412 A